# EUROPEAN PATENT APPLICATION

(11) **EP 4 279 081 A1**
(43) Date of publication of application: **22.11.2023**
(21) Application number: 21927342.2
(22) Date of filing: 01.03.2021
(51) Int. Cl.: A61K 38/17, A61K 9/06, A61K 47/42, A61P 19/00, A61P 19/08, A61K 35/28

(54) **USE OF POLYPEPTIDE IN PROMOTION OF CARTILAGE REGENERATION OR REPAIR**

(30) Priority: 25.02.2021 CN 202110214216
(71) Applicant: Hangzhou Huibo Science and Technology Co., Ltd, Hangzhou, Zhejiang 311217 (CN)
(72) Inventor: WU, Gang, Hangzhou, Zhejiang 311217 (CN); WU, Liyong, Hangzhou, Zhejiang 311217 (CN); FENG, Jianying, Hangzhou, Zhejiang 311217 (CN); SHI, Changjin, Hangzhou, Zhejiang 311217 (CN); DU, Yiyang, Hangzhou, Zhejiang 311217 (CN); LU, Yunyu, Hangzhou, Zhejiang 311217 (CN); YAO, Yu, Hangzhou, Zhejiang 311217 (CN); GUO, Jing, Hangzhou, Zhejiang 311217 (CN)
(74) Representative: Longland, Emma Louise
(86) International application number: PCT/CN2021/078448
(87) International publication number: WO 2022/178907

(57) **Abstract**

Disclosed is use of a Histatin-1 polypeptide in promotion of cartilage regeneration or repair. By preparing a combined reagent containing a Histatin-1 polypeptide, a biological scaffold material and the like, mainly a linear Hst1 is loaded on gelatin methacrylate so as to be used for repairing cartilage damage or promoting cartilage growth; meanwhile, disclosed is use of a Histatin-1 polypeptide in promotion of the expression of one or more of bone differentiation indexes, including a collagen fiber, glycosaminoglycan, type II collagen and aggrecan.

## Description

### BACKGROUND OF THE INVENTION

### Field of the Invention

The present invention relates to the technical field of biomedicine, in particular to the field of cartilage regeneration or repair, and especially to use of a Histatin-1 polypeptide in promotion of cartilage regeneration or repair.

### Description of the Related Art

Cartilage is composed of a cartilage tissue and its surrounding perichondrium, wherein the cartilage tissue is composed of a chondrocyte, a matrix and a fiber. The cartilage is for example articular cartilage or temporomandibular joint (TMJ) cartilage.

The articular cartilage is a thin layer of connective tissues with high water content, having no nerves, having no blood vessels and with viscoelasticity, which covers a surface of a joint to provide lubricative joint movement and transfers a mechanical force to a subchondral bone. According to the morphology of the cartilage and the running form of collagen, the articular cartilage shows a typical layered structure. A surface layer is characterized by slender and flat chondrocytes with parallel and thin collagen fibers. An intermediate layer contains round chondrocytes and randomly oriented thick collagen fibers. In contrast, a deep layer has round and vertically arranged chondrocytes, and thick collagen fibers orthogonal to a contacting surface. A calcified layer has round and hypertrophic chondrocytes and obvious mineralized collagen I. The calcified layer is connected with a bone tissue.

The osteochondral defect of a temporomandibular joint (TMJ) may be caused by acute injury of condyle, overload or an abnormal immune response. Even some daily activities (such as talking, eating and yawning) may cause pain and limited mandibular movement, which will plague a patient with condylar osteochondral defects for all his/her life.

Due to the limited self-repair potential of the cartilage, it is very challenging to repair osteochondral defects in the TMJ. Firstly, the avascular characteristic of a condylar cartilage tissue leads to inability to produce a classic healing response, such as coagulation, inflammation, blood invasion and aggregation of multifunctional mesenchymal stem cells (MSCs). Secondly, the migration and proliferation of chondrocytes around the defect region are poor. This causes the TMJ cartilage to have almost no self-repair ability. Thirdly, when the defect further expands deeply to a subchondral bone tissue, the blood supply from a bone tissue will trigger the classical healing response and enhance the migration of MSCs to some extent. However, the blood supply and migration of MSCs from the subchondral bone are limited, which is not enough to completely repair the osteochondral defects. Meanwhile, the osteochondral defects reduce the soft surface area of condyle, which leads to mechanical overload of other TMJ tissues, thereby causing secondary mechanical damage to TMJ. Currently, in clinic, the osteochondral defects are mainly repaired by using autologous transplantation, such as autologous chondrocyte implantation and mosaicplasty. These repairing approaches provide chondrocytes for the defect region, and have certain effects in the repair of the osteochondral defects. However, due to the limited sampling site and size of a autologous graft, postoperative pain occurs in a donor site and a series of complications such as arthropathies may be caused, and thus the use of the autologous transplantation method is greatly limited. Therefore, it has always been a hot spot in the field of TMJ repair to find a best method for repairing the osteochondral defects.

Cartilage tissue engineering combines the biomaterial scaffold with a bioactive agent or a stem cell, and a resultant composite material is applied for tissue reconstruction and has broad prospects. The scaffold is an important integral part of tissue engineering, which provides support for cell migration and new tissue generation in the defect region.

Carrying the mesenchymal stem cells (MSCs) or the bioactive agent through the biomaterial scaffold is expected to promote complete repair of the osteochondral defects, but tissue engineering technology carrying the bioactive agent has a series of advantages, such as low cost, wide sources and simple and convenient operation, as compared with carrying the MSCs. Therefore, it is urgent to find a more ideal bioactive agent, which can induce angiogenesis and in vivo migration of the MSCs from a bone defect region to a cartilage lesion site, thereby promoting the repair of the osteochondral defects.

A Histatin is a group of histidine-rich cationic polypeptides secreted by a parotid gland and a submandibular gland of human, and has a length of 7-38 amino acid residues. Histatins 1, 3 and 5 are the 3 most important ones. In recent years, the Histatin-1 polypeptide has been deeply studied in the field of biomedicine, which has functions of promoting cell adhesion, extension, migration, angiogenesis, anti-inflammation and the like functions.

CN107108751A discloses that the histatin can be used for treating tooth demineralization and assisting in realizing remineralization of an enamel surface; in the patent application CN108785657A filed by the inventor of the present application in 2018, disclosed is use of the Histatin-1 polypeptide in preparation of a composite material for promoting the repair of largearea skin defects; however, there is no related report about the use of the Histatin-1 polypeptide in promoting the repair of the osteochondral defects or promoting cartilage growth.

### BRIEF SUMMARY OF THE INVENTION

In view of the problems existed in the prior art, the present invention provides a Histatin-1 polypeptide which can be used for preparing a formulation for repairing cartilage damage or promoting cartilage growth and a related composition thereof, and discloses that the composition has the efficacy of promoting expression of type II collagen and/or Aggrecan.

In an aspect, the present invention provides use of the Histatin-1 polypeptide in preparation of a reagent for promoting cartilage regeneration or repair.

In the prior art, it is believed that the Histatin-1 (Hst1) polypeptide has the ability of promoting the adhesion and migration of epithelial cells, fibroblasts and osteoblasts. Meanwhile, it can promote cell metabolic activity and maintain cell viability in various unfavorable environments. However, these functions are essentially different from promoting cartilage repair and cartilage growth.

The cartilage described in the present invention includes all cartilage tissues, such as articular cartilage or temporomandibular joint (TMJ) cartilage, etc.

The repair of a cartilage injury or defect needs to guide homing of stem cells, chondrogenic differentiation of stem cells, proliferation and hypertrophy of chondrocytes, etc. Mesenchymal stem cells have multipotential differentiation directions, such as directions of osteogenesis, chondrogenesis, myogenesis and adipogenesis, etc. Runx2, Sox9, MyoD and PPARγ are key transcription factors in these directions, respectively, which induce the expression of respective downstream pathways. However, the same stem cell can only differentiate in one direction, and high expression of one transcription factor will inhibit the expression of other transcription factors, thereby inhibiting other differentiation directions. Among these factors, the key transcription factor of osteogenic differentiation is RUNX2, which protein is a member of Runx transcription factor family and has a Runt DNA binding domain. This is very important for osteoblast differentiation and bone morphogenesis. It acts as a scaffold for nucleic acids and regulatory factors involved in bone gene expression. The protein can be either used as a monomer, or as a subunit of a heterodimer complex to bind DNA. Transcript variants of genes encoding different protein isoforms are produced by using alternative promoters and alternative splicing. The cyto-dynamics of the Runx2 protein is also important for proper osteoblast differentiation. The Runx2 protein is detected in osteoblasts, and its expression is up-regulated in immature osteoblasts, but down-regulated in mature osteoblasts. It is the first transcription factor required for determining osteoblast commitment, followed by Sp7 and Wnt signaling. Runx2 is responsible for inducing pluripotent mesenchymal cells to differentiate into immature osteoblasts, and activating the expression of several key downstream proteins that maintain osteoblast differentiation and bone matrix genes. Knockout of DNA binding activity leads to inhibition of osteoblast differentiation. Therefore, Runx2 is usually referred to as a main regulator of a bone. BMP-2 can directly up-regulate Runx2 through a p-Smad signaling pathway, and in turn induce osteogenic differentiation.

Although the Histatin-1 polypeptide has the ability of promoting the adhesion and migration of epithelial cells, fibroblasts and osteoblasts. Meanwhile, it can promote cell metabolic activity and maintain cell viability in various unfavorable environments. However, it has been found by researches of the present invention that, the polypeptide can promote the repair or growth of cartilage injury, and its mechanism of action may be one or comprehensive reflection of multiple ones of the following: firstly, the Histatin-1 polypeptide promotes angiogenesis, which is relatively important for a bone defect. Cartilage needs blood vessels to deliver nutrients and stem cells, but there should be no blood vessels in the cartilage, so that the mechanism of promoting blood vessels by Hst1 is more to assist chondrogenesis than to directly induce cartilage regeneration. Secondly, a cartilage island mainly appears around a cartilage wall in an early stage, so that the Histatin-1 polypeptide may promote chondrocytes to express a large number of factors for promoting cartilage differentiation (e.g., Runx2, Sox9, MyoD, PPARγ and the like factors), thereby inducing stem cells into cartilage differentiation as guided by the Histatin-1 polypeptide. Thirdly, it has also been found in the present invention that the Histatin-1 polypeptide significantly promotes the expression of a cartilage-related protein, such as type II collagen and/or Aggrecan; and thus it indicates that the Histatin-1 polypeptide can significantly promote high expression of an osteogenic differentiation index.

Further, the Histatin-1 polypeptide can promote the expression of the osteogenic differentiation marker.

Further, the osteogenic differentiation marker includes a collagen fiber and/or glycosaminoglycan.

Studies have proved that the Histin-1 polypeptide can increase the percentage of the area of one or more of a new subchondral bone, a collagen fiber of cartilage and glycosaminoglycan (GAG) in the total area of the defect region.

Further, the osteogenic differentiation marker further includes the type II collagen and/or Aggrecan.

Studies have proved that the Histatin-1 polypeptide can promote cartilage regeneration or repair by promoting the expression of the type II collagen and/or Aggrecan.

Further, the Histatin-1 polypeptide is derived from a polypeptide isolated from saliva, or an artificially synthesized polypeptide.

In some embodiments, the Histatin-1 polypeptide may be derived from a polypeptide isolated from saliva; or alternatively, the Histatin-1 polypeptide can of course be synthesized artificially.

Further, the artificially synthesized polypeptide is a linear Hst1.

Further, the linear Hst1 has an amino acid sequence as shown in SEQ ID NO.1 of Sequence Listing.

The artificially synthesized polypeptide may be an artificially synthesized linear Hst1, of which the amino acid sequence is:
DSHEKRHHGYRRKFHEKHHSHREFPFYGDYGSNYLYDN (SEQ ID No.1).

Further, the Histatin-1 polypeptide needs to be combined with a biological scaffold material into a composition for use.

Further, the biological scaffold material includes one or more of gelatin, collagen protein, hyaluronic acid, chitosan, sodium alginate, heparin, polyvinylalcohol, dextran, carboxymethyl cellulose, ethylene glycol chitosan, propylene glycol chitosan, chitosan lactate, carboxymethyl chitosan, chitosan quaternary ammonium salt, hydrophilic or water-soluble animal and plant proteins, collagen, a serum protein, two-arm or multi-arm polyethylene glycol, polyethyleneimine, a dendrimer, a synthetic polypeptide, polylysine or (meth)acrylate or (meth)acrylamide, and modification products of the aforementioned ingredients.

A biological scaffold reagent is generally a reagent carrying an active ingredient, which can be safe with a biological tissue, or keep the release and slow release of the active ingredient, so that the active ingredient can exert its greater or better physiological functions. In some embodiments, the biological scaffold reagent may be some safe biological materials with good biocompatibility, such as natural polysaccharides (e.g., hyaluronic acid, heparin, alginic acid, dextran, carboxymethyl cellulose, ethylene glycol chitosan, propylene glycol chitosan, chitosan lactate, carboxymethyl chitosan, chitosan quaternary ammonium salt, etc.) and modification or degradation products thereof; it can also be a protein or polypeptide, such as various hydrophilic or water-soluble animal and plant proteins, collagen, serum proteins, gelatin, and modification products and/or degraded polypeptides thereof, etc.; and it can also be a hydrophilic or water-soluble synthetic polymer, such as two-arm or multi-arm polyethylene glycol, polyethyleneimine, a dendrimer, a synthetic polypeptide, polylysine or (meth)acrylate or (meth)acrylamide, or other polymer molecules, etc.

Further, the biological scaffold material is methacrylate gelatin.

Methacrylate gelatin (Gel-MA) has a similar chemical composition to collagen and has good biocompatibility. Unlike collagen, the Gel-MA has no risk of pathogen transmission. Moreover, the Gel-MA contains a large number of adhesion ligands, such as an arginine-glycine-aspartic acid sequence, so as to promote cell adhesion and migration.

The Gel-MA has good flowability before curing, which makes it flexible to match a defect region of complex cavity type; and after photo-curing, the Gel-MA converts from a liquid form to a hydrogel form, and its hardness reaches 50-60 KPa, which is beneficial to the formation of cartilage and bone tissues.

By using the Gel-MA as the biological scaffold and carrying the Histin-1 polypeptide as a bioactive agent, it can effectively promote the repair of osteochondral defects.

Further, in the composition, the mass ratio of the Histatin-1 polypeptide to methacrylate gelatin is 2.5-50:211, or 50-1,000 µg of the Histatin-1 polypeptide is loaded on 21.1 µl of methacrylate gelatin.

Further, in the composition, the mass ratio of the Histatin-1 polypeptide to methacrylate gelatin is 25:211, or 500 µg of the Histatin-1 polypeptide is loaded on 21.1 µl of methacrylate gelatin.

Further, the composition further includes a photoinitiator.

The photoinitiator can stabilize a formed gel structure by modifying the biological scaffold reagent, and can assist in realizing the treatment of cartilage injury and tissue repair, for example using an o-nitrobenzyl photoresponsive group for modification and conducting light irradiation, or using hyaluronic acid modified by N-(2-aminoethyl)-4-(4-(hydroxymethyl)-2-methoxy-5-nitrophenoxy)butyramide mixed with chitosan, sodium alginate, polyvinylalcohol, etc.

Further, the photoinitiator is 2-hydroxy-1-(4-(hydroxyethoxy)phenyl)-2-methyl-1-acetone.

Further, the composition further includes a stem cell.

The composition further includes other auxiliary reagents, such as the stem cell, etc.

A method for preparing the composition reagent for promoting cartilage regeneration or repair of the present invention includes the following steps: dissolving 200 mg of a lyophilized Gel-MA macromolecular polymer in 1 ml of PBS, and filtering with a bacterial filter at 80°C to obtain a Gel-MA hydrogel prepolymer solution; and taking and dissolving 50-1,000 µg of a Histatin-1 polypeptide in 21.1 µl of the Gel-MA hydrogel prepolymer solution, and meanwhile adding a proper amount of an auxiliary reagent such as a photoinitiator.

In another aspect, the present invention provides use of a Histatin-1 polypeptide in preparation of a reagent for promoting the expression of one or more of a collagen fiber, glycosaminoglycan, type II collagen and Aggrecan.

It has been proven through a large number of studies in the present invention that, the Histatin-1 polypeptide can promote the increase of collagen fiber area and the increase in expression of glycosaminoglycan, type II collagen and Aggrecan.

Further, the Histatin-1 polypeptide is derived from a polypeptide isolated from saliva, or an artificially synthesized polypeptide.

Further, the artificially synthesized polypeptide is a linear Hst; and the linear Hst1 has an amino acid sequence as shown in SEQ ID NO.1 of Sequence Listing.

Further, the Histatin-1 polypeptide needs to be combined with a biological scaffold material into a composition for use.

Further, the biological scaffold material is methacrylate gelatin.

Further, in the composition, the mass ratio of the Histatin-1 polypeptide to methacrylate gelatin is 2.5-50:211, or 50-1,000 µg of the Histatin-1 polypeptide is loaded on 21.1 µl of methacrylate gelatin.

Further, in the composition, the mass ratio of the Histatin-1 polypeptide to methacrylate gelatin is 25:211, or 500 µg of the Histatin-1 polypeptide is loaded on 21.1 µl of methacrylate gelatin.

Further, the composition further includes a stem cell and/or a photoinitiator.Beneficial effects of the present invention are as follows.
1. Disclosed is the use of a Histatin-1 polypeptide in preparation of a reagent for repairing cartilage injury or promoting cartilage growth; and a combined reagent for repairing cartilage injury or promoting cartilage growth and a preparation method thereof are provided.
2. It is disclosed that the Histatin-1 polypeptide can be used for preparing a reagent for promoting the expression of one or more of a collagen fiber, glycosaminoglycan, type II collagen and Aggrecan, and a combined reagent for promoting the expression of one or more of a collagen fiber, glycosaminoglycan, type II collagen and Aggrecan and a preparation method thereof are provided.

### BRIEF DESCRIPTION OF THE DRAWINGS

Fig. 1 is a schematic diagram of the preparation of a rabbit temporomandibular joint condylar osteochondral defect (3 mm*3 mm) model of Example 2.
Fig. 2 is a photograph of the rabbit condylar osteochondral defect of Example 2 and a comparison diagram of macroscopic evaluation results.
Fig. 3 is an optical photomicrograph of a H&E stained tissue section of the rabbit condyle at 4 weeks after operation in Example 3.
Fig. 4 shows quantitative analysis of the areas of new cartilage and subchondral bone in Example 3, and is a comparison diagram of MODS score results of repairing osteochondral defects at 1, 2 and 4 weeks after operation.
Fig. 5 is a comparison diagram of special staining results of repaired osteochondral defects in a Gel-MA group and a Hst1/Gel-MA group in Example 3.
Fig. 6 is a comparison diagram of the results of quantitative analysis of glycosaminoglycan (GAG) and collagen fibers in the defect region, new cartilage and new subchondral bone at 2 and 4 weeks after operation in Example 3.
Fig. 7 is an optical photomicrograph of immunohistochemically stained tissue sections of type II collagen and aggrecan in the Gel-MA group and the Hstl/Gel-MA group in Example 3 and a comparison diagram of quantitative analysis of their expression.
Fig. 8 is a comparison diagram of optical photomicrographs of H&E stained tissue sections of the rabbit condyle at different Hst1 concentrations in Example 4.

### DETAILED DESCRIPTION OF THE INVENTION

The present invention will be further described in detail with reference to examples, and it should be pointed out that the following examples are intended to facilitate the understanding of the present invention, without any limitation to it.

### Example 1 Preparation of Gel-MA hydrogel prepolymer solution and Hst1

Gel-MA lyophilized powder used in this example was purchased from Wenzhou Institute (Wenzhou Institute, Wenzhou, China).

200 mg of the lyophilized Gel-MA macromolecular polymer was dissolved in 1 ml of PBS containing 0.5% (w/v) of 2-hydroxy-1-(4-(hydroxyethoxy)phenyl)-2-methyl-1-acetone (photoinitiator 2959, CIBA Specialty Chemicals, Basel, Switzerland, with a concentration of 50 mg/ml, added at 0.42 µl), and filtered with a bacterial filter at 80°C to obtain a prepolymer solution. The prepolymer solution was stored in a constant temperature water bath tank at 40°C. A fresh hydrogel prepolymer solution was prepared before operation and kept in a sterile flask.

The lyophilized linear Hst1 polypeptide was available from Amsterdam university (Amsterdam, the Netherlands, with the amino acid sequence as shown in SEQ ID NO.1), and was stored in an environment of -20°C. 500 µg of the lyophilized Hst1 polypeptide was dissolved in 21.1 µl (the volume of the defect region) of the Gel-MA prepolymer solution to prepare an implantation reagent.

### Example 2 Macroscopic evaluation of HST1 in promoting cartilage repair

54 adult male New Zealand white rabbits (Zhejiang Chinese Medical University, IACUC-20180625-04) were randomized into 3 groups, with 18 rabbits in each group. The 54 rabbits were operated at right condyle, while the left condyle remained intact.

They were divided into 3 groups, of which group 1 was a control group without any treatment in the defect region; group 2 was a Gel-MA group implanted with Gel-MA hydrogel in the defect region; and group 3 was a Hst1/Gel-MA group implanted with HST1 functionalized Gel-MA in the defect region (provided in Example 1).

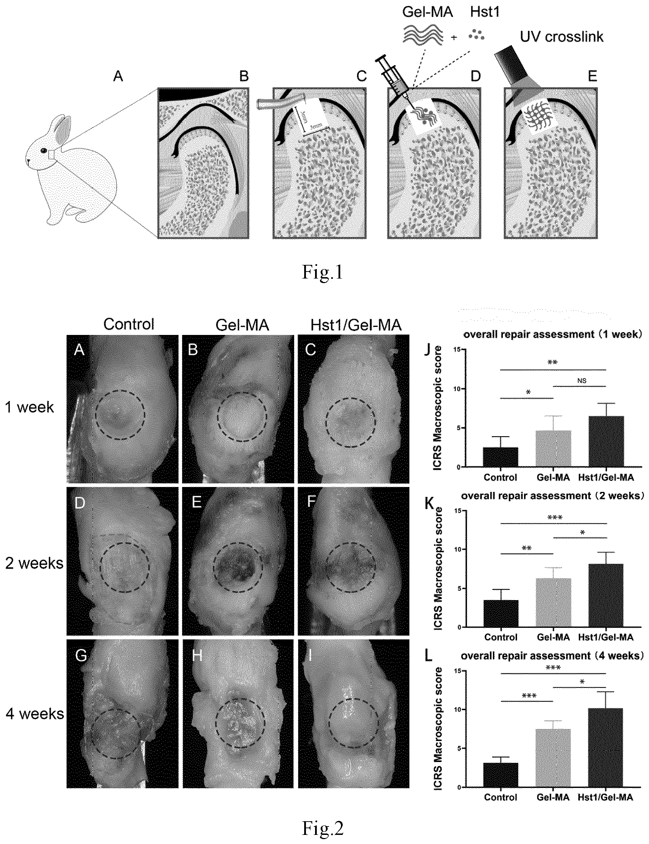

After general anesthesia of the rabbit, a preauricular skin incision was made in the right temporomandibular joint to expose a condylar head, and an osteochondral defect with a diameter of 3 mm and a depth of 3 mm was made on the condyle by using a drill bit with a diameter of 3 mm (as shown in A-C of Fig. 1).

The group 1 was the control group in which the defect region was prepared without further treatment.

In the group 2, the Gel-MA hydrogel was injected into the defect region.

In the group 3, the HST1 functionalized Gel-MA was injected into the defect site (as shown in D of Fig. 1).

The Gel-MA and a prepolymer solution thereof in the groups 2 and 3 were photopolymerizated with ultraviolet light (365 nm, 90 s) (as shown in E of Fig. 1). The wound was sewed in layers with a nylon suture. After all the steps were completed, the animals were sent back to the animal feeding center, and intraperitoneally injected with penicillin (1: 100,000) every 24 hours over first three days after operation.

At 1, 2 and 4 weeks after operation, 6 animals in each group were randomly selected for euthanasia. The right temporomandibular joint and surrounding tissues thereof were taken out, and the gross specimens were evaluated by macroscopic scoring, and statistically analyzed.

The macroscopic scoring evaluation of the gross specimens adopted a macroscopic scoring system of the International Cartilage Repair Society (ICRS), and the gross appearance of the defect site was photographed and subjected to blind evaluation by3 independent graders. The scoring system evaluated the cartilage repair in the defect region from four aspects: the degree of repair, the integration of a boundary area, a macroscopic appearance and the overall repair evaluation. The evaluation criteria were shown in Table 1.

**Table 1. Macroscopic Scoring Criteria for Gross Specimens**

| Classification | Index parameter | Score |
|---|---|---|
| Degree of defect repair | Same as surrounding normal cartilage | 4 |
| | Repaired 75% of the depth of defect | 3 |
| | Repaired 50% of the depth of defect | 2 |
| | Repaired 25% of the depth of defect | 1 |
| | No repair | 0 |
| Integration of boundary | There is no obvious boundary, and it is completely integrated with the surrounding cartilage | 4 |
| | Boundary < 1 mm | 3 |
| | 75% of it was integrated, and 25% of it had a significant gap of > 1 mm | 2 |
| | 50% of it was integrated, and 50% of it had a significant gap of > 1 mm | 1 |
| | With a degree of integration less than 25% | 0 |
| General observation of appearance | The surface was smooth and complete | 4 |
| | The surface was fibrous | 3 |
| | Small cracks or scars could be seen on the surface | 2 |
| | A few large cracks could be seen on the surface | 1 |
| | No repair, or complete degeneration | 0 |
| | Overall repair score: grade I: completely normal | 12 |
| | grade II: good repair | 8-11 |
| | grade III: there was repair, but the effect was not good | 4-7 |
| | grade IV: there was no repair effect | 0-3 |

Statistical analysis was conducted by performing one-way ANOVA and a Tukey's HSD post-hoc test with SPSS 18.0 statistical analysis software (SPSS Inc, USA), and all data were expressed as mean ± standard deviation (SD). P < 0.05 indicated that the difference was statistically significant, and P < 0.01 and 0.001 indicated that the difference was highly statistically significant.

All rabbits recovered well after operation and could uptake enough food every day to maintain their original weights. No complications occurred at 4 weeks after operation. All rabbits survived until they were executed.

Tissues were taken at 1, 2 and 4 weeks after operation, and the photographs and macroscopic evaluation results of rabbit condylar osteochondral defect were shown in Fig. 2, in which a red circle area represented an original defect region and a blue irregular area represented an expanded absorption area. The group 1 as the control group was shown as A, D and G in Fig. 2. The group 2 as the Gel-MA group was shown as B, E and H in Fig. 2. The group 3 as the Hst1/Gel-MA group was shown as C, F and I in Fig. 2. The macroscopic scoring results obtained by ICRS were shown as J-L in Fig. 2, which represented the scoring results of the sample at 1, 2 and 4 weeks respectively. (n = 6, **P < 0.01; ***P < 0.001).

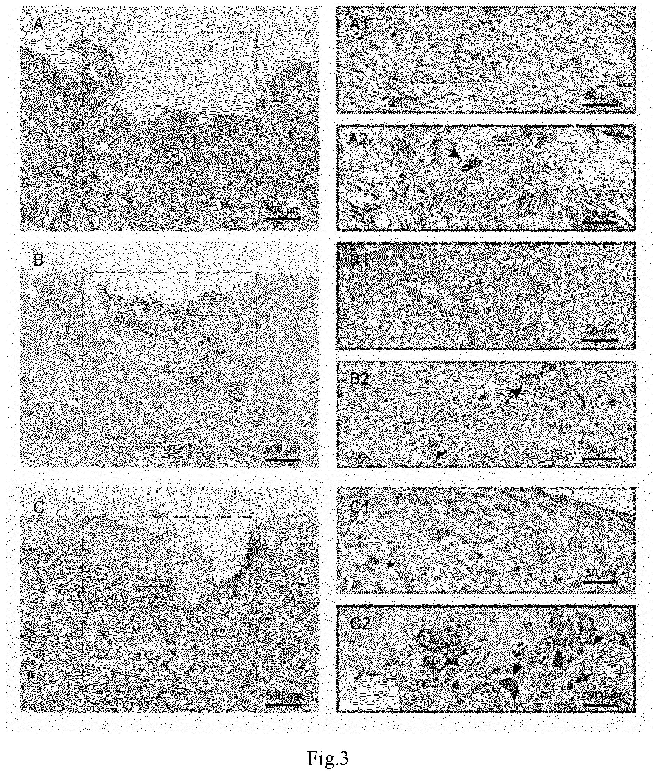

As could be seen from Fig. 2, in the group 3 as the Hst1/Gel-MA group, the boundary of tissue between the defect region and the surrounding normal cartilage tissues was obvious at 1 week after operation, and only 25% of the defect boundary was integrated (Fig. 2C); at 2 weeks, the boundary between the tissue in the defect region and the surrounding cartilage became blurred, 50% of the defect boundary was integrated, and the new tissue repaired 50% of the depth of the defect (Fig. 2F); and at 4 weeks, the defect had no obvious boundary and was completely integrated with the surrounding cartilage, an inner surface of the defect region was smooth and intact, and the color and texture of the new tissue were similar to those of the surrounding normal cartilage, and 75% of the depth of the defect was repaired (Fig. 2I). In contrast, the defect in the group 2 were not fully filled with a reddish tissue, and there were still depressions on the surface of the defect at 4 weeks (Fig. 2H). In the group 1, diffuse degenerative changes were observed at 4 weeks (Fig. 2G). During the 1 to 4 weeks, the ICRS macroscopic scoring of the group 3 was significantly higher than those of the groups 2 and 1. At the same time point, the difference among the groups was statistically significant (p < 0.05) (Fig. 3, J-L). It could be seen that Hst1 had a very significant effect on promoting the repair of cartilage injury.

### Example 3 Histological evaluation of HST1 in promoting cartilage repair and promoting the expression of collagen fiber, glycosaminoglycan (GAG), type II collagen and Aggrecan

In this example, 3 groups of rabbits provided in Example 2 were used, and at 1, 2 and 4 weeks after operation, 6 animals in each group were randomly selected for euthanasia. The right temporomandibular joint and surrounding tissues thereof were taken out, and the gross specimens were analyzed for tissue morphology and statistically analyzed.

Histological examination steps were as follows: in an environment of 4°C, the specimens used for histological analysis were fixed in 10% neutral formalin (Sig-ma-Aldrich, USA) for 24 hours, and decalcified with a 10% ethylenediaminetetraacetic acid (EDTA) buffer solution (Sigma-Aldrich, USA) for 28 days. The sample was embedded in paraffin wax (Sigma-Aldrich, USA), cut into 4 µm thick sagittal sections, and subjected to staining with hemotoxylin and eosin, Masson trichrome and Alcian Blue (Sigma Aldrich, USA). The sections were detected immunohistochemically for the contents of the type II collagen and Aggrecan. All the sections were observed and photographed by utilizing an optical microscope (CX51; Olympus) and a digital charge coupled device camera (TrueChrome Metrics; Tucsen Photonics). These sections were scored blindly by 3 experienced pathologists with an improved O'Driscoll scoring system (MODS).

The steps of histological morphology analysis were as follows: in all the sample sections, the original defect region was a 3 * 3 mm square. The top of the square was flush with the cartilage surfaces on both sides. The percentages of areas of the new subchondral bone, the cartilage, the collagen fiber and the glycosaminoglycan (GAG) in the total area of the defect region was detected with image analysis software (image pro plus 6.0). Their respective contents were expressed as integrated optical density (IOD) of the type II collagen and Aggrecan measured by Image pro plus 6.0.

At 4 weeks after operation, the tissues were retrieved for histological treatment and sectioning. The optical photomicrographs of H&E stained tissue sections of rabbit condyle were shown in Fig. 3. According to the implants in the defect region, they were divided into a group 1 as the control group (A in Fig. 3, A1-A2), a group 2 as the Gel-MA group (B in Fig. 3, B1-B2) and a group 3 as the Hst1/Gel-MA group (C in Fig. 3, C1-C2). The dotted square area in Fig. 3 was the original defect region (3 mm * 3 mm). A-C scale = 500 µm. A1: the surface of the defect was covered with fibrous tissues; A2: osteoclasts could be seen between subchondral bones in the defect region; B1: it was found that fibrous tissues crawling between undegraded scaffold materials in the defect region; B2: there was subchondral osteogenesis in the defect region; C1: chondrocytes were arranged in layers above the defect region; and C2: there was subchondral osteogenesis in the defect region, with infiltrating of a large number of osteoblasts and osteoclasts. Solid arrow: osteoclasts; hollow arrow: osteoblasts; pentagram: immature chondrocytes; and triangle: new blood vessels. A1-C2 scale: 50 µm.

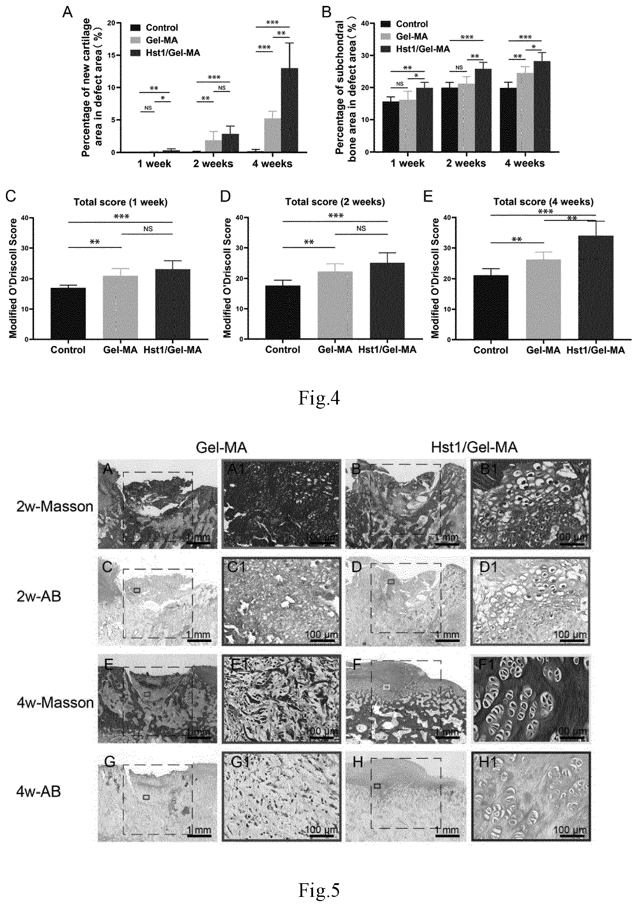

The quantitative analysis of areas of new cartilage and subchondral bones and the MODS score results of repairing osteochondral defects at 1, 2 and 4 weeks after operation were shown in Fig. 4. In Fig. 4, A was the quantitative analysis of the area of the new cartilage, B was the quantitative analysis of the area of the subchondral bone, and C, D and E were the MODS score results of repairing osteochondral defects at 1, 2 and 4 weeks after operation, respectively, (n = 6, P < 0.05, P < 0.01, P < 0.001).

As could be seen from A and B of Fig. 4, at 1, 2 and 4 weeks, the area percentage of the new subchondral bone and cartilage in the defect region of the group 3 was significantly higher than those of the groups 2 and 1 (P < 0.05) (A-B of Fig. 4). Moreover, at 1, 2 and 4 weeks, the MODS score of the group 3 was significantly higher than those of groups 1 and 2. At the same time point, there was a statistical difference in the scores among the groups (C-E in Fig. 4). It could be seen that the Hst1/Gel-MA group could significantly promote the repair of the subchondral bone and cartilage.

At 1 week, a new tissue was observed in the pores of the undegraded scaffold in the group 3. At 2 weeks, new cartilage and bone tissues gradually grew, infiltrated and replaced the scaffold. A new cartilage island could be seen in the undegraded scaffold. At 4 weeks, the new cartilage proliferated and migrated to the center site of the defect. Moreover, as could be seen from C, C1 and C2 in Fig. 3, the new chondrocytes presented a typical cartilage structure and were arranged in columns (C1 in Fig. 3); there was a fibrous layer above the new chondrocyte layer, which was similar to a fibrous layer in normal mandibular condylar cartilage (C1 in Fig. 3); at the bottom of the defect, the reconstruction of a subchondral bone was observed with infiltrating of a large number of osteoblasts and osteoclasts (C2 in Fig. 3); and the formation of new blood vessels could be detected at a junction between the subchondral bone and a functionalized hydrogel (C2 in Fig. 3). Meanwhile, as could be seen from Fig. 3, compared with the group 3, only a small amount of cell infiltration could be detected in the undegraded scaffold in the group 2 at 4 weeks. The defect region was filled with a large amount of fibrous tissues and partial of undegraded scaffold materials (B-B1 in Fig. 3). In the group 1, the defect region was still in a hollow state, and only the bottom of the defect region was covered with one layer of fibrous tissues (A-A1 in Fig. 3). Therefore, compared with the control group, Hst1 had a very significant effect on promoting the repair of cartilage injury.

The special staining and quantitative analysis of the repaired osteochondral defects in the group 2 as the Gel-MA group and the group 3 as the Hstl/Gel-MA group were shown in Fig. 5, wherein Masson trichrome staining was performed at 2 weeks (A and B in Fig. 5) and 4 weeks (E and F in Fig. 5) after operation. Alcian Blue staining was performed at 2 weeks (C and D in Fig. 5) and 4 weeks (G and H in Fig. 5) after operation. A-H scale = 1 mm; and A1-H1 scale = 100 µm.

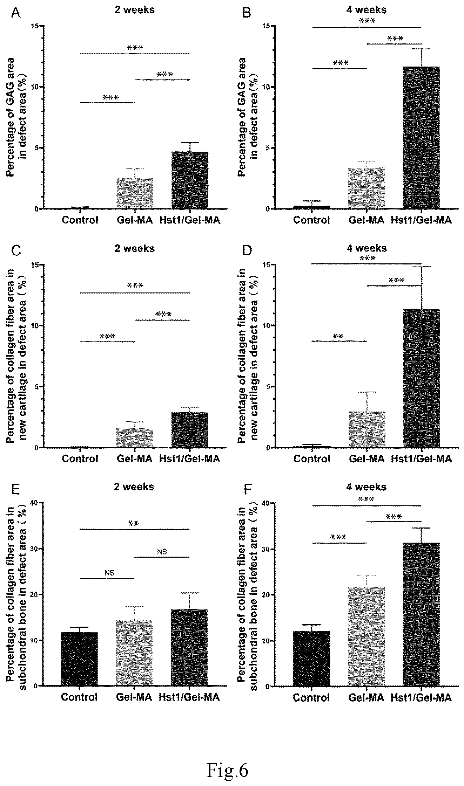

The defect region, the new cartilage and the new subchondral bone were quantitatively analyzed for glycosaminoglycan (GAG) and collagen fibers at 2 and 4 weeks after operation, and the results were shown in Fig. 6, wherein A and B in Fig. 6 were the GAG area percentages in the defect region at 2 and 4 weeks, C and D were the collagen fiber area percentages in the new cartilage at the 2 and 4 weeks, and E and F were the collagen fiber area percentages in the subchondral bone at the 2 and 4 weeks. (n = 6, **P < 0.01, ***P <0.001, NS = not significant).

As could be seen from Figs. 5 and 6, in the group 3, as confirmed by Alcian Blue staining and Masson trichrome staining at 2 and 4 weeks (A-H1 in Fig. 5), the area percentages of the collagen fibers in the new cartilage and the subchondral bone in the total area of the defect region and the area percentages of glycosaminoglycan (GAG) in the new cartilage and the subchondral bone in the total area of the defect region were significantly higher than those in the groups 2 and 1 (P < 0.05) (Fig. 6, A-F). Therefore, compared with the control group, Hst1 could significantly promote the expression of the collagen fibers and glycosaminoglycan (GAG) in the subchondral bone and cartilage.

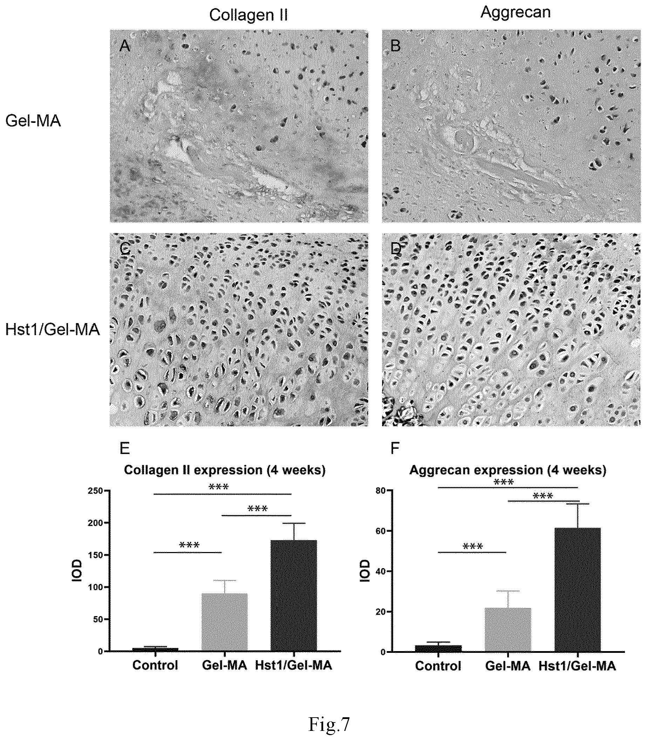

At 2 and 4 weeks after operation, the tissues were taken out, sectioned and subjected to immunohistochemical treatment. The photographs of tissue sections immunohistochemically stained for the type II collagen and Aggrecan in the Gel-MA group and the Hst1/Gel-MA group under an optical microscope were shown in Fig. 7, wherein the group 2 as the Gel-MA group was shown in A and B of Fig. 7, the group 3 as the Hst1/Gel-MA group was shown in C and D of Fig. 7, the results of the type II collagen were shown in A and C of Fig. 7, and the results of the Aggrecan were shown in B and D of Fig. 7; and a A-D scale was: 100 µm. (n = 6, ***P < 0.001); and the expression of the type II collagen (E in Fig. 7) and Aggrecan (F in Fig. 7) were quantitatively analyzed at 4 weeks after operation.

As could be seen from Fig. 7, in the group 3, the expression of the type II collagen and Aggrecan was increased gradually over time after operation. At 4 weeks after operation, the expression of the type II collagen and Aggrecan in the new cartilage tissue was close to those in the normal cartilage tissue (C-D in Fig.7). However, in the group 2, at 1 and 2 weeks, almost no type II collagen and Aggrecan were detected in the repaired tissues. At 4 weeks, staining of the collagen II and Aggrecan was observed (A-B in Fig. 7). In the group 1, no positive immunostaining results of the type II collagen and Aggrecan was observed at all time points. At 4 weeks, compared with the groups 2 and 1, the expression of the type II collagen and Aggrecan in the group 3 was more significant (E-F in Fig. 7). Therefore, it could be seen obviously from Fig. 7 that Hst1 could significantly promote the expression of the type II collagen and Aggrecan.

### Example 4 Effect of Proportional Relationship between Gel-MA Hydrogel and Hst1 on Promoting Cartilage Repair

In order to determine the optimal concentration of Hst1 for promoting osteochondral repair, Gel-M and 3 different doses of Hst1 at (A, A1) 50, (B, B1) 500, and (C, C1) 1,000 (µg)/sample were implanted in the defect region of a critical size in rabbit condyle, and the tissues were taken out at 2 weeks after operation, subjected to histological treatment and sectioned to explore the cartilage repair effects of the 3 different concentrations of Hst1.

The 3 different concentrations of Hst1 were set as follows: group A: 50 µg of Hst1 + 21.1 µl of a Gel-MA prepolymer solution; group B: 500 µg of Hst1 + 21.1 µl of a Gel-MA prepolymer solution; and group C: 1,000 µg of Hst1 + 21.1 µl of a Gel-MA prepolymer solution (the model volume of condylar defect was 21.1 µl/sample).

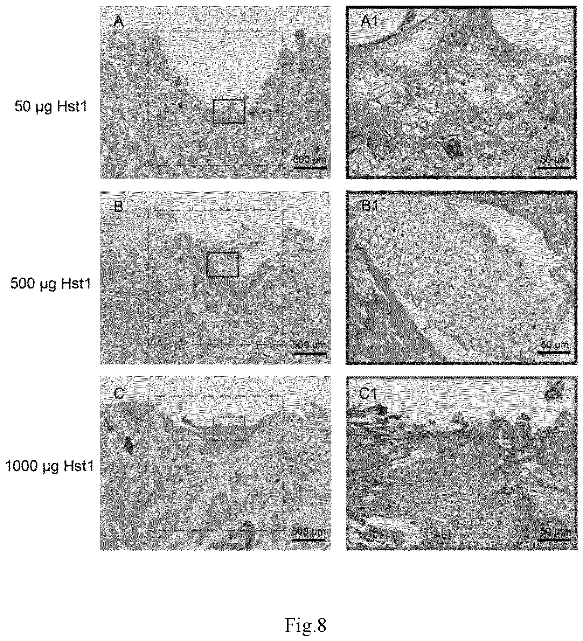

The optical photomicrographs of H&E stained tissue sections of rabbit condyle for comparison were shown in Fig. 8, wherein the dotted square area was the original defect region (3 mm * 3 mm), the A-C scale was 500 µm; and the A1-C1 scale = 50 µm. The results of Fig. 8 showed that at 2 weeks, several new cartilage islands could be obviously observed in the group B (Fig. 8, B-B1); a large number of new subchondral bones were generated, but almost no new cartilage tissue was detected in the defect region of the group C (Fig. 8, C-C1); and no new tissue was generated, and only a small amount of cell infiltration was detected in the undegraded scaffold at the bottom of the defect region in the group A (Fig. 8, A-A1). Therefore, 500 µg of Hst1/sample (500 µg of Hst1 + 21.1 µl of Gel-MA) was selected as the preferred concentration.

Although the present invention is disclosed above, the present invention is not limited thereto. Various changes and modifications can be made by those of skills in the art without departing from the spirit and scope of the present invention, and thus the claimed scope of the present invention should be based on the scope defined by the claims.

### Sequence list

SEQ ID NO.1
a linear Hst1:
DSHEKRHHGYRRKFHEKHHSHREFPFYGDYGSNYLYDN

## Claims

1. Use of a Histatin-1 polypeptide in preparation of reagent for promoting cartilage regeneration or repair damaged cartilage.

2. The use according to claim 1, wherein the Histatin-1 polypeptide can promote the expression of an osteogenic differentiation marker.

3. The use according to claim 2, wherein the osteogenic differentiation marker comprises a collagen fiber and/or glycosaminoglycan.

4. The use according to claim 2 or 3, wherein the osteogenic differentiation marker comprises a type II collagen and/or Aggrecan.

5. The use according to claim 4, wherein the Histatin-1 polypeptide is isolated from saliva, or an artificially synthesized.

6. The use according to claim 5, wherein the artificially synthesized polypeptide is a linear Hst1.

7. The use according to claim 6, wherein the linear Hst1 has an amino acid sequence as shown in SEQ ID NO.1 of Sequence Listing.

8. The use according to claim 7, wherein the reagent further comprises a biological scaffold material which forms a composition withthe Histatin-1 polypeptide.

9. The use according to claim 8, wherein the biological scaffold material comprises one or more of gelatin, collagen protein, hyaluronic acid, chitosan, sodium alginate, heparin, polyvinylalcohol, dextran, carboxymethyl cellulose, ethylene glycol chitosan, propylene glycol chitosan, chitosan lactate, carboxymethyl chitosan, chitosan quaternary ammonium salt, hydrophilic or water-soluble animal and plant proteins, collagen, a serum protein, two-arm or multi-arm polyethylene glycol, polyethyleneimine, a dendrimer, a synthetic polypeptide, polylysine or (meth)acrylate or (meth)acrylamide, and modification products of the aforementioned ingredients.

10. The according to claim 9, wherein the biological scaffold material is methacrylate gelatin.

11. The use according to claim 10, wherein in the composition, the mass ratio of the Histatin-1 polypeptide to methacrylate gelatin is 2.5-50:211, or 50-1,000 µg of the Histatin-1 polypeptide is loaded on 21.1 µl of methacrylate gelatin.

12. The use according to claim 11, wherein in the composition, the mass ratio of the Histatin-1 polypeptide to methacrylate gelatin is 25:211, or 500 µg of the Histatin-1 polypeptide is loaded on 21.1 µl of methacrylate gelatin.

13. The use according to any one of claims 8-12, wherein the composition further comprises a photoinitiator and/or a stem cell.

14. The use according to claim 13, wherein the photoinitiator is 2-hydroxy-1-(4-(hydroxyethoxy)phenyl)-2-methyl-1-acetone.

15. Use of an Histatin-1 polypeptide for promoting a expression of one or more of a collagen fiber, glycosaminoglycan, type II collagen and Aggrecan.

16. The use according to claim 15, wherein the Histatin-1 polypeptide is derived from a polypeptide isolated from saliva, or an artificially synthesized polypeptide.

17. The use according to claim 16, wherein the artificially synthesized polypeptide is a linear Hst; and the linear Hst1 has an amino acid sequence as shown in SEQ ID NO.1 of Sequence Listing.

18. The use according to claim 17, wherein the Histatin-1 polypeptide is needed to be combined with a biological scaffold material into a composition for use.

19. The use according to claim 18, wherein the biological scaffold material is methacrylate gelatin.

20. The use according to claim 19, wherein in the composition, the mass ratio of the Histatin-1 polypeptide to methacrylate gelatin is 25:211, or 500 µg of the Histatin-1 polypeptide is loaded on 21.1 µl of methacrylate gelatin.
